# EUROPÄISCHE PATENTSCHRIFT

(11) **EP 1 611 863 B1**
(45) Veröffentlichungstag und Bekanntmachung des Hinweises auf die Patenterteilung: **18.02.2009**
(21) Anmeldenummer: 04015308.2
(22) Anmeldetag: 30.06.2004
(51) Int. Cl.: A61B 19/00

(54) **Erzeugung eines dreidimensionalen Körperteilmodells aus Fluoroskopiebilddaten und speziellen Landmarken**
Generation of a three-dimensional model of a body portion based on fluoroscopic image data and specific landmarks
Génération d'un modèle d'une partie de corps à trois dimensions fondée sur des données d'image fluoroscopique et des indices spécifiques

(43) Veröffentlichungstag der Anmeldung: 04.01.2006
(73) Patentinhaber: BrainLAB AG, 85622 Feldkirchen (DE)
(72) Erfinder: Grünschläger, Frank, 85622 Feldkirchen (DE); Haimerl, Martin, 82205 Gilching (DE); Lachner, Rainer, 85586 Poing (DE); Vilsmeier, Stefan, 6330 Kufstein (AT); Ritter, Alf, 85253 Kleinberghofen (DE)
(74) Vertreter: Schwabe - Sandmair - Marx

(56) Entgegenhaltungen:
- EP-A- 1 348 394
- DE-A- 10 037 491

## Beschreibung

Die vorliegende Erfindung betrifft ein Verfahren zur Erzeugung eines dreidimensionalen Körperteilmodells mit Hilfe einer medizinischen bzw. chirurgischen Navigationseinrichtung. Insbesondere betrifft sie die Erzeugung eines solchen Modells ohne eine vorangehende Schichtbilderfassung, also insbesondere ohne vorangehende Computertomographie. Noch spezieller betrifft die vorliegende Erfindung die Erzeugung eines dreidimensionalen Körperteilmodells, das für Hüftimplantations-Operationen geeignet ist.

Derzeit verwendete Techniken zur CT-freien Navigation umfassen hauptsächlich die Navigation mit Hilfe von Röntgenbildern unter Verwendung von Fluoroskopie-Geräten. Dabei werden mittels des Fluoroskopie-Gerätes Bilder akquiriert, worauf nach Kalibrierung und einer Verzerrungskorrektur Landmarken in den Bildern bestimmt werden. Diese reine Fluoroskopie-Navigation ist umständlich und erfordert oftmals die Erstellung vieler Röntgenaufnahmen, um alle notwendigen Daten zu jedem gewünschten Zeitpunkt parat zu haben. Dies bringt eine erhöhte Strahlenbelastung für den Patienten aber auch für das Operationspersonal mit sich.

Die EP 1 329 202 B1 beschreibt ein Verfahren und eine Einrichtung zur Zuordnung einer digitalen Bildinformation zu den Navigationsdaten eines medizinischen Navigationssystems, wobei insbesondere mit einem digitalen C-Bogen-Röntgengerät erstellte Bilddaten in die Navigation eingebunden werden. Auch bei dieser Technik müssen in Abfolge immer wieder Röntgenaufnahmen gemacht werden, was eine entsprechende Strahlenbelastung mit sich bringt.

Die EP 1 348 394 offenbart ein Verfahren, bei dem ein dreidimensionales generisches Modell durch eine Datenverknüpfung auf zweidimensionaler Ebene mit patientencharacteristischen zweidimensionalen Erfassungsdaten angepasst wird, um dreidimensionale Korperstrukturdaten zu erhalten.

Es ist die Aufgabe der vorliegenden Erfindung, ein Verfahren zur Erzeugung eines dreidimensionalen Körperteilmodells zu schaffen, welches die Nachteile des Standes der Technik überwindet. Insbesondere soll mit einfachen Mitteln eine dreidimensionale Navigation ermöglicht werden, ohne dass vorab Schichtbilderfassungen, speziell CT-Aufnahmen gemacht werden müssen. Speziell soll es möglich sein, ein ausreichend gutes, dreidimensionales Körperteilmodell zu erstellen, mit dessen Hilfe navigiert werden kann, ohne dass in der Abfolge andauernd neue Fluoroskopieaufnahmen gemacht werden müssen.

Diese Aufgabe wird durch ein Verfahren gemäß dem Patentanspruch 1 gelöst. Die Unteransprüche definieren bevorzugte Ausführungsformen des erfindungsgemäßen Verfahrens.

Die Vorteile der vorliegenden Erfindung beruhen darauf, dass bei einem erfindungsgemäßen Verfahren zur Erzeugung eines dreidimensionalen Körperteilmodells mit Hilfe einer medizinischen bzw. chirurgischen Navigationseinrichtung die folgenden Schritte durchgeführt werden:
- für das Körperteilmodell charakteristische Landmarken werden mit einem navigierten Zeigegerät abgegriffen und positionell bestimmt;
- mindestens zwei Fluoroskopiebilddatensätze werden jeweils für eine oder mehrere vorbestimmte, einzelne und abgegrenzte Körperteilregion erstellt;
- charakteristische Körperteildaten werden aus einer computergestützten Verarbeitung und Kombination der Informationen, nämlich aus den unmittelbar ermittelten Landmarkenpositionen und aus Kenngrößen, die mittels Bildverarbeitung aus den Fluoroskopiedatensätzen gewonnen werden, ermittelt; und
- sein aus den charakteristischen Körperteildaten wird ein dreidimensionales und positionell bestimmtes Körperteilmodell für die bildunterstützte, chirurgische Navigation erstellt.

Das erfindungsgemäße Verfahren bedient sich also nicht lediglich der relativ aufwändigen Auswertung von Fluoroskopiebilddatensätzen, sondern es werden zusätzlich noch spezielle und charakteristische Landmarken abgegriffen, wodurch sich die Erzeugung des Körperteilmodells sehr viel einfacher und auch rechnerisch weniger aufwändig gestaltet als wenn lediglich Fluoroskopiebilddatensätze ausgewertet werden. Die abgegriffenen Landmarken geben absolute Raumpunkte wieder, die das Erstellen des gesamten Modells mit Hilfe der Fluoroskopiedaten einfacher gestalten. Mit anderen Worten mischt die vorliegende Erfindung zwei voneinander unabhängig durchführbare Arten der Erfassung von Körpermerkmalen, nämlich die Definition spezieller Landmarken und die Erstellung von Fluoroskopiebilddatensätzen in einer solchen Weise, dass eine dreidimensionale Modellerstellung einerseits möglich und andererseits einfach und schnell durchführbar wird. Wenn für bestimmte, einzelne und abgegrenzte Körperteilregionen jeweils zwei Fluoroskopiebilddatensätze aus unterschiedlichen Erfassungsrichtungen erstellt werden, wird die dreidimensionale Modellerstellung in einfacher Weise ermöglicht. Außerdem können sich die auf unterschiedliche Weise akquirierten Positionsdaten gegenseitig synergetisch ergänzen, da nicht durch das Zeigegerät abgreifbare Punkte, also beispielsweise weitere Landmarken, durch Symmetrieberechnung an symmetrisch oder im Wesentlichen symmetrisch ausgebildeten Körperteilen bestimmt werden können, wenn Informationen aus fluoroskopischen Durchleuchtungsbildern hierzu verwendet werden.

Die charakteristischen Körperteildaten können Längen von Körperteilabschnitten und Winkel von Körperteilabschnitten zueinander umfassen.

Erfindungsgemäß können auch zusätzlich Gelenkrotations-Mittelpunkte bestimmt werden, indem charakteristische Landmarken oder eine Navigation-Referenzanordnung an einem beweglichen Gelenkknochen an mehreren Gelenkwinkelpositionen positionell bestimmt werden und dann aus den erhaltenen Bahnpunkten auf das Rotationszentrum zurückgerechnet wird.

Bei einer speziellen Ausführungsform der vorliegenden Erfindung wird ein Körperteilmodell eines Oberschenkelknochens erzeugt, und dabei werden folgende Schritte durchgeführt:
- folgende Landmarken werden mit dem navigierten Zeigegerät abgegriffen und positionell bestimmt:
   -- Epicondylus lateralis (femoris),
   -- Epicondylus medialis (femoris);
- der Gelenkrotations-Mittelpunkt des Oberschenkelknochens wird bestimmt durch:
   -- positionelle Bestimmung einer der obigen Landmarken oder einer Navigations-Referenzanordnung an mehreren Gelenkwinkelpositionen und durch Zurückrechnen auf den Mittelpunkt aus den erhaltenen Bahnpunkten; und/oder
   -- eine Bildverarbeitung unter Verwendung von Fluoroskopiebildern die in verschiedenen Gelenkwinkelpositionen aufgenommen werden;
- die mechanische Achse des Oberschenkelknochens wird bestimmt durch eine Verbindung des Gelenkrotations-Mittelpunktes mit dem Mittelpunkt zwischen Epicondylus lateralis und Epicondylus medialis;
- die anatomische Achse des Oberschenkelknochens wird bestimmt durch eine Bildverarbeitung unter Verwendung von Fluoroskopiebildern des Knochenschaftbereichs, bei der mehrere Achsenpunkte in der Knochenmitte bestimmt werden, die dann den Verlauf der anatomischen Achse bestimmen;
- die Halsachse wird bestimmt durch
   -- eine Bildverarbeitung unter Verwendung von zwei Fluoroskopiebildern des Oberschenkelhalsbereichs; und/oder
   -- eine Bildverarbeitung unter Verwendung von eines Fluoroskopiebildes des Oberschenkelhalsbereichs, und unter Verwendung der Position des Gelenkrotations-Mittelpunktes und einer bekannten bzw. vorbestimmten windschiefen Lage der Halsachse zur anatomischen Achse; und/oder
   -- das Abgreifen und die positionelle Bestimmung von Punkten bzw. Mittelpunkten am Oberschenkelhals;
- die Antetorsion wird aus schon ermittelten Informationen bestimmt, insbesondere aus der Lage der Verbindung zwischen Epicondylus lateralis und Epicondylus medialis und der Lage der mechanischen Achse.

Ferner kann gemäß der vorliegenden Erfindung das Verfahren ein Körperteilmodell eines Beckens erzeugen, wobei folgende Schritte durchgeführt werden:
- die Lage von mindestens einer Spina iliaca anterior superior wird mit dem navigierten Zeigegerät abgegriffen und positionell bestimmt;
- die sagittale Mittelebene des Beckens wird bestimmt durch eine Bildverarbeitung unter Verwendung von Fluoroskopiebildern eines symmetrischen Beckenstrukturbereichs und der Symmetrieeigenschaften des Beckens, wobei auch die Lage der zweiten Spina iliaca anterior superior ermittelt werden kann;
- die frontale Beckenebene wird bestimmt durch eine Lageermittlung für den vorderen Schambeinpunkt mittels Bildverarbeitung unter Verwendung von Fluoroskopiebildern des Schambeinfugenbereichs und durch Verbindung des Punktes mit den ermittelten Punkten für die Spina iliaca anterior superior.

Das Körperteilmodell kann bei einer bevorzugten Ausführungsform des erfindungsgemäßen Verfahrens mit Hilfe von generischen Körperteildaten ergänzt oder vervollständigt werden, insbesondere durch Verwendung eines generischen Körperteilmodells, welches anhand schon ermittelter Informationen für das zu erzeugende Körperteilmodell angepasst wurde.

Gemäß einer weiteren Ausführungsvariante des erfindungsgemäßen Verfahrens wird vor dem Abgreifen einer Landmarke bzw. vor der Erstellung der Fluoroskopiebilddatensätze jeweils an einer Bildausgabe angezeigt, welche Landmarke im Ablauf als nächstes abgegriffen werden soll bzw. welches Fluoroskopiebild im Ablauf als nächstes erstellt werden soll.

Die Erfindung wird im Weiteren anhand möglicher Ausführungsformen und mit Hilfe der beiliegenden Zeichnungen näher erläutert. Die hierin beschriebenen Merkmale der vorliegenden Erfindung können einzeln und in jedweder Kombination umgesetzt werden. In den beiliegenden Zeichnungen zeigen:
- Figur 1: eine schematische Darstellung von Oberschenkel- und Beckenknochen mit der Benennung wichtiger Merkmale;
- Figur 2: eine Schemadarstellung entsprechend Figur 1 mit zusätzlich angezeigten Körperteilregionen für Fluoroskopie-Aufnahmen; und
- Figur 3: einen Screenshot eines Programms für die Unterstützung bei der Aufnahme von Fluoroskopiebilddatensätzen.

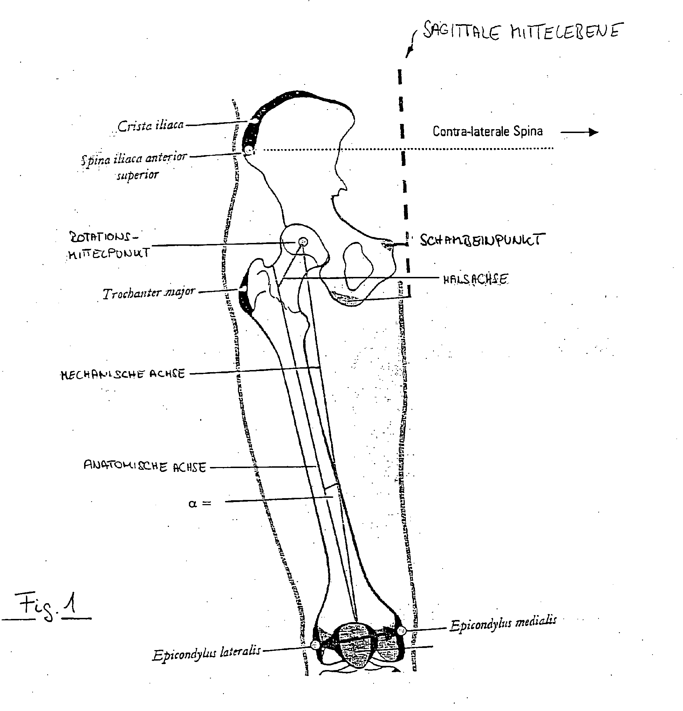

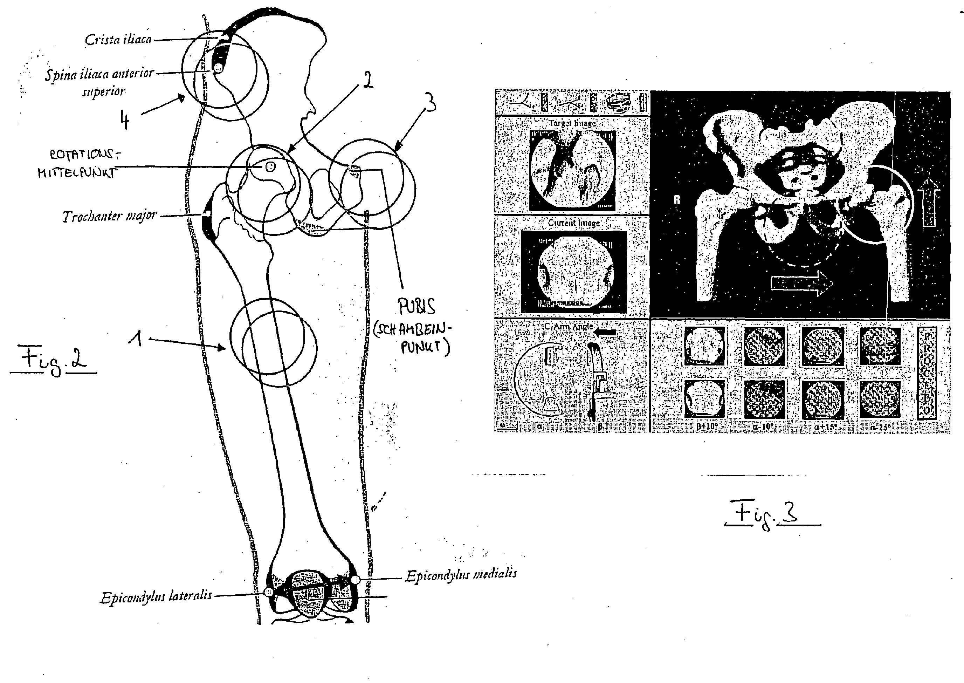

Die Erfindung soll nun anhand einer Modellerzeugung näher beschrieben werden, wie sie beispielsweise bei Hüftoperationen zur Anwendung kommen kann. In den Figuren 1 und 2 sind die Merkmale ersichtlich, auf die im Weiteren Bezug genommen wird.

Es soll die Möglichkeit geschaffen werden, anhand eines 3D-Modells zu navigieren, das aus zweidimensionalen fluoroskopischen Bildaufnahmen und speziellen Landmarken generiert wird, wobei die dreidimensionale Ausrichtung des menschlichen Beckenknochens verbessert werden soll, um eine geeignete Implantatpositionierung bei chirurgischen Gesamt-Hüftersatz-Prozeduren zu ermöglichen. Dies umfasst nicht nur den Hüftknochen sondern auch den Oberschenkelknochen.

Bei der Pfannenimplantat-Positionierung sind zwei Ausrichtungsparameter wichtig: Die Pfannen-Anteversion und die Pfannen-Inklination. Diese beiden Winkel beziehen sich auf ein Hüftkoordinatensystem, welches durch die Anatomie des Hüftknochens definiert wird, nämlich durch die frontale Beckenebene und eine sagittale Mittelebene. Diese beiden Ebenen stellen die Basis für alle Winkelberechnungen zum Setzen des Pfannenimplantats dar, um das anatomische Hüftgelenk zu ersetzen.

Für das Setzen des Oberschenkelknochenimplantats werden ebenfalls zwei relevante Ausrichtungsparameter definiert, nämlich die Schaftachse und die Halsachse (Oberschenkelhalsachse). Diese beiden Achsen stellen die Basis für alle Berechnungen zum Setzen des Schaft- bzw. Oberschenkelknochenimplantats beim Ersatz des anatomischen Hüftgelenkes dar.

Gemäß der vorliegenden Erfindung werden spezielle Landmarken mit einem Navigations-Pointer (Zeigegerät) des Navigationssystems akquiriert und diese Informationen werden kombiniert mit Informationen, die aus Fluoroskopiebildern erhalten werden, beispielsweise durch Bildverarbeitungsalgorithmen. In Figur 1 sind unter anderem diejenigen Punkte angedeutet, die mittels des Pointers akquiriert werden, während in Figur 2 mit dem Bezugszeichen 1 bis 4 jeweils Bereiche bzw. Regionen angedeutet sind, für die Fluoroskopieaufnahmen erstellt werden. Die beiden jeweils für eine Region dargestellten Kreise deuten an, dass immer zwei Fluoroskopieaufnahmen für jede Region aus verschiedenen Winkeln erstellt werden, so dass eine dreidimensional Rekonstruktion der dabei erfassten Merkmale ermöglicht wird. Die Bildverarbeitung kann dabei entweder mittels des Fluoroskopiegerätes selbst oder durch eine Bildverarbeitungseinheit des Navigationssystems durchgeführt werden.

Die Bestimmung des Koordinatensystems des Beckens ist dann schwierig, wenn spezielle Landmarken nicht körperlich selbst, also beispielsweise durch den Navigationpointer akquiriert werden können. Dann muss auf Fluoroskopiebilder zurückgegriffen werden, welche die Knochenstruktur (Becken- oder Oberschenkelknochen) beschreiben. Hierbei können Symmetrieeigenschaften der Knochenstrukturen ausgenützt werden. Beispielsweise kann die sagittale Mittelebene des Beckens bestimmt werden, ohne dass ein Pointer-Zugriff auf beide Spina iliaca anterior superior-Punkte besteht, nämlich einfach dadurch, dass Röntgenaufnahmen des Tuberculum-Pubis-Bereichs gemacht werden. Ein automatischer Algorithmus kann dann die Mittel-Symmetrieachse des Bildes errechnen, und ein anderer automatischer Algorithmus errechnet die Ausrichtung der Mittel-Symmetrieebene. Dies ist ebenfalls bei der Oberschenkel-Schaftachse und der Halsachse möglich.

Relativ einfach kann meist ein Spina iliaca anterior superior-Punkt durch einen Pointer abgegriffen werden, dies gilt ebenso für Epycondylus lateralis und Epicondylus medialis am Oberschenkelknochen. Der Rotations-Mittelpunkt für den Oberschenkelknochen lässt sich dadurch errechnen, dass diese Landmarken oder eine Referenzanordnung am Knochen bei einem Verschwenken des Knochens im Navigationssystem getrackt bzw. positionell verfolgt werden. Die Punkte werden sich dann auf einer Kugeloberfläche bewegen und diese Oberfläche definiert in ihrem Mittelpunkt den Rotations-Mittelpunkt. Per Software kann dann auch eine mechanische Achse definiert werden, die durch zwei Punkte bestimmt wird, nämlich durch den Rotations-Mittelpunkt und die Mitte der epicondularen Achse.

Nachdem diese Vorregistrierung abgeschlossen ist, werden acht Fluoroskopiebilder, nämlich vier Paare von Fluoroskopiebildern in spezifischen und abgegrenzten Regionen erstellt, die in Figur 2 mit den Bezugszeichen 1 bis 4 bezeichnet sind. Die speziellen Regionen für die Fluoroskopiebilder sind: proximaler Oberschenkelknochen 1, Oberschenkelhals 2, Pubis (Schambein) 3 und Spina iliaca anterior superior 4. Für jede Region sind zwei Fluoroskopiebilder zu machen, um aus den zweidimensionalen Bildern dreidimensionale Informationen zu erhalten.

Die Bildinformationen aus dem Pubisbereich gestatten unter anderem die Ermittlung der sagittalen Mittelebene, die Berechnung des kontralateralen Spina-Punktes und die Ermittlung der frontalen Beckenebene.

Die Bildinformationen für das Acetabulum/Oberschenkelhals gestatten unter anderem die Ermittlung der Halsachse mittels aktiver Konturen, die Verwendung eines Vorgabewertes für die Halsachse aufgrund des Winkels zwischen Hals und Schaft (ungefähr 130°), die automatische Ermittlung des Oberschenkelkopfes (Pfannengröße ist gleich Kopfdurchmesser plus acht Millimeter) und eine LL-Berechnung aus Pfannenposition und Oberschenkelimplantat.

Die Bilddaten für den proximalen Oberschenkelknochen ermöglichen unter anderem die Erfassung der Oberschenkel-Schaftachse (anatomische Achse mittels aktiver Konturen), der Winkel zwischen anatomischer und mechanischer Achse kann mit 7° vorausgesetzt werden, und mit diesen Informationen lässt sich die Größe des Oberschenkelimplantats bestimmen.

Die Bildinformationen im Bereich der Spina iliaca anterior superior lassen unter anderem die automatische Erfassung der Crista iliaca zu.

Mit den so gewonnenen Informationen lässt sich also grundsätzlich schon ein dreidimensionales Körperteilmodell für Becken und Oberschenkelknochen einstellen, welches einerseits die Navigation ermöglicht und andererseits die Planung für das Setzen des Implantats gestattet.

Sehr einfach gestaltet sich die richtige Akquirierung von Fluoroskopiebildern, wenn sie durch einen Software-Assistenten unterstützt wird. Dieser Assistent führt das Behandlungsteam durch die Akquirierung der Fluoroskopiebilder, und er ist in verschiedene Abschnitte unterteilt. Der Hauptteil des Assistenten besteht aus einem Modell mit zwei Kreisen, die beispielsweise in dem Screenshot der Figur 3 erkennbar sind. Der gestückelte Kreis beschreibt die tatsächliche Position des C-Bogen-Fluoroskopiegerätes, das hierfür durch das verwendete Navigationssystem getrackt werden kann. Der durchgezogene Kreis zeigt die Zielposition, um ein Fluoroskopiebild einer speziellen Region zu erstellen. Im unteren Teil der Darstellung zeigt eine Statusanzeige an, welche Bilder schon akquiriert werden und welche noch zu erstellen sind.

Links oben wird die Sichtbarkeit von Referenzanordnungen der verwendete Werkzeuge durch Farbmarkierungen angezeigt. Ein Zielbild hilft dabei, das beste Bild für eine spezielle Region zu finden. Diese Bilder können mit dem darunter gezeigten, gerade aktuellen Bild verglichen werden. Der letzte Sektor links unten zeigt den Winkel des C-Bogens an.

Mit Hilfe eines solchen Assistenten gestaltet sich die Akquirierung der Fluoroskopiebilder einfach und schnell. Da auch die Akquirierung der Landmarkenpositionen durch den Pointer sehr schnell vonstatten gehen kann, lässt sich durch die vorliegende Erfindung in einfacher und schneller Weise ein dreidimensionales Körperteilmodell erzeugen, welches dann, möglicherweise ergänzt durch angepasste generische Daten, der Planung und Navigation des chirurgischen Eingriffs zugrundegelegt werden kann.

## Patentansprüche

1. Verfahren zur Erzeugung eines dreidimensionalen Körperteilmodells mit Hilfe einer medizinischen bzw. chirurgischen Navigationseinrichtung und mit den folgenden Schritten:
- für das Körperteilmodell charakteristische Landmarken werden mit einem navigierten Zeigegerät abgegriffen und positionell bestimmt;
- mindestens zwei Fluoroskopiebilddatensätze werden jeweils für eine oder mehrere vorbestimmte, einzelne und abgegrenzte Körperteilregionen erstellt;
- charakteristische Körperteildaten werden aus einer computerunterstützten Verarbeitung und Kombination der Informationen, nämlich aus den unmittelbar ermittelten Landmarkenpositionen und aus Kenngrößen, die mittels Bildverarbeitung aus den Fluoroskopiedatensätzen gewonnen werden, ermittelt; und
- rein aus den charakteristischen Körperteildaten wird ein dreidimensionales und positionell bestimmtes Körperteilmodell für die bildunterstützte, chirurgische Navigation erstellt.

2. Verfahren nach Anspruch 1, bei dem die charakteristischen Körperteildaten weitere Landmarken umfassen, die durch Symmetrieberechnung an symmetrisch oder im Wesentlichen symmetrisch ausgebildeten Körperteilen bestimmt werden.

3. Verfahren nach Anspruch 1 oder 2, bei dem die charakteristischen Körperteildaten Längen von Körperteilabschnitten und Winkel von Körperteilabschnitten zueinander umfassen.

4. Verfahren nach einem der Ansprüche 1 bis 3, bei dem zusätzlich Gelenkrotations-Mittelpunkte bestimmt werden, indem charakteristische Landmarken oder eine Navigations-Referenzanordnung an einem beweglichen Gelenkknochen an mehreren Gelenkwinkelpositionen positionell bestimmt werden, und dann aus den erhaltenen Bahnpunkten auf das Rotationszentrum zurückgerechnet wird.

5. Verfahren nach einem der Ansprüche 1 bis 4, bei dem ein Körperteilmodell eines Oberschenkelknochens erzeugt wird, und bei dem einer oder mehrere der folgenden Schritte durchgeführt werden:
- folgende Landmarken werden mit dem navigierten Zeigegerät abgegriffen und positionell bestimmt:
-- Epicondylus lateralis,
-- Epicondylus medialis;
- der Gelenkrotations-Mittelpunkt des Oberschenkelknochens wird bestimmt durch:
-- positionelle Bestimmung einer der obigen Landmarken oder einer Navigations-Referenzanordnung an mehreren Gelenkwinkelpositionen und durch Zurückrechnen auf den Mittelpunkt aus den erhaltenen Bahnpunkten; und/oder
-- eine Bildverarbeitung unter Verwendung von Fluoroskopiebildern die in verschiedenen Gelenkwinkelpositionen aufgenommen werden;
- die mechanische Achse des Oberschenkelknochens wird bestimmt durch eine Verbindung des Gelenkrotations-Mittelpunktes mit dem Mittelpunkt zwischen Epicondylus lateralis und Epicondylus medialis;
- die anatomische Achse des Oberschenkelknochens wird bestimmt durch eine Bildverarbeitung unter Verwendung von Fluoroskopiebildern des Knochenschaftbereichs, bei der mehrere Achsenpunkte in der Knochenmitte bestimmt werden, die dann den Verlauf der anatomischen Achse bestimmen;
- die Halsachse wird bestimmt durch
-- eine Bildverarbeitung unter Verwendung von zwei Fluoroskopiebildern des Oberschenkelhalsbereichs; und/oder
-- eine Bildverarbeitung unter Verwendung von eines Fluoroskopiebildes des Oberschenkelhalsbereichs, und unter Verwendung der Position des Gelenkrotations-Mittelpunktes und einer bekannten bzw. vorbestimmten windschiefen Lage der Halsachse zur anatomischen Achse; und/oder
-- das Abgreifen und die positionelle Bestimmung von Punkten bzw. Mittelpunkten am Oberschenkelhals;
- die Antetorsion wird aus schon ermittelten Informationen bestimmt, insbesondere aus der Lage der Verbindung zwischen Epicondylus lateralis und Epicondylus medialis und der Lage der mechanischen Achse.

6. Verfahren nach einem der Ansprüche 1 bis 5, bei dem ein Körperteilmodell eines Beckens erzeugt wird, und bei dem folgende Schritte durchgeführt werden:
- die Lage von mindestens einer Spina iliaca anterior superior wird mit dem navigierten Zeigegerät abgegriffen und positionell bestimmt;
- die sagittale Mittelebene des Beckens wird bestimmt durch eine Bildverarbeitung unter Verwendung von Fluoroskopiebildern eines symmetrischen Beckenstrukturbereichs und der Symmetrieeigenschaften des Beckens, wobei auch die Lage der zweiten Spina iliaca anterior superior ermittelt werden kann;
- die frontale Beckenebene wird bestimmt durch eine Lageermittlung für den vorderen Schambeinpunkt mittels Bildverarbeitung unter Verwendung von Fluoroskopiebildern des Schambeinfugenbereichs und durch Verbindung des Punktes mit den ermittelten Punkten für die Spina iliaca anterior superior.

7. Verfahren nach einem der Ansprüche 1 bis 6, bei dem das Körperteilmodell mit Hilfe von generischen Körperteildaten ergänzt oder vervollständigt wird, insbesondere durch Verwendung eines generischen Körperteilmodells, welches anhand schon ermittelter Informationen für das zu erzeugende Körperteilmodell angepasst wurde.

8. Verfahren nach einem der Ansprüche 1 bis 7, bei dem vor dem Abgreifen einer Landmarke bzw. vor der Erstellung der Fluoroskopiebilddatensätze jeweils an einer Bildausgabe angezeigt wird, welche Landmarke im Ablauf als nächste abgegriffen werden soll bzw. welches Fluoroskopiebild im Ablauf als nächstes erstellt werden soll.

## Claims

1. A method for generating a three-dimensional model of a part of the body with the aid of a medical and/or surgical navigation means, and comprising the following steps:
- landmarks which are characteristic of the model of the part of the body are tapped and positionally determined using a navigated pointer;
- at least two fluoroscopy image data sets are produced for each of one or more predetermined, individual and delimited regions of the part of the body;
- characteristic body part data are ascertained from processing and combining the information with computer assistance, i.e. from the directly ascertained landmark positions and from parameters obtained from the fluoroscopy data sets by means of image processing; and
- a three-dimensional and positionally determined model of the part of the body for image-assisted, surgical navigation is produced purely from the characteristic body part data.

2. The method according to claim 1, wherein said characteristic body part data include other landmarks which are determined by a symmetry calculation on symmetrically or substantially symmetrically formed parts of the body.

3. The method according to claim 1 or 2, wherein the characteristic body part data include lengths of body part sections and angles of body part sections to each other.

4. The method according to any one of claims 1 to 3, wherein joint rotation centre points are in addition determined by positionally determining characteristic landmarks or a navigation reference array on a movable joint bone at a number of joint angular positions and then calculating back to the rotational centre from the trajectory points obtained.

5. The method according to any one of claims 1 to 4, wherein a model of a part of the body - of a femur - is generated, and wherein the following steps are performed:
- the following landmarks are tapped and positionally determined using the navigated pointer:
- epicondylus lateralis;
- epicondylus medialis;
- the joint rotation centre point of the femur is determined by:
- positionally determining one of the above landmarks or a navigation reference array at a number of joint angular positions and calculating back to the centre point from the trajectory points obtained; and/or
- image processing using fluoroscopy images recorded in various joint angular positions;
- the mechanical axis of the femur is determined by connecting the joint rotation centre point and the centre point between the epicondylus lateralis and epicondylus medialis;
- the anatomical axis of the femur is determined by image processing using fluoroscopy images of the bone shaft region, wherein a number of axis points in the centre of the bone are determined which then determine the course of the anatomical axis;
- the neck axis is determined by:
- image processing using two fluoroscopy images of the region of the neck of the femur; and/or
- image processing using one fluoroscopy image of the region of the neck of the femur and using the position of the joint rotation centre point and a known and/or predetermined skewed position of the neck axis relative to the anatomical axis; and/or
- tapping and positionally determining points and/or centre points on the neck of the femur;
- antetorsion is determined from information already obtained, in particular from the position of the connection between the epicondylus lateralis and epicondylus medialis and the position of the mechanical axis.

6. The method according to any one of claims 1 to 5, wherein a model of a part of the body - of a pelvis - is generated, wherein the following steps are performed:
- the position of at least one spina iliaca anterior superior is tapped and positionally determined using the navigated pointer;
- the mid-sagittal plane of the pelvis is determined by image processing using fluoroscopy images of a symmetrical pelvic structure region and the symmetry properties of the pelvis, wherein the position of the second spina iliaca anterior superior can also be ascertained;
- the frontal pelvic plane is determined by ascertaining the position for the front point of the pubic bone by means of image processing using fluoroscopy images of the pubic symphysis region and by connecting the point to the points ascertained for the spina iliaca anterior superior.

7. The method according to any one of claims 1 to 6, wherein the model of the part of the body is supplemented or completed with the aid of generic body part data, in particular by using a generic model of the part of the body which has been adapted on the basis of information already ascertained for the model of the part of the body to be generated.

8. The method according to any one of claims 1 to 7, wherein an image output displays - before a landmark is tapped or the fluoroscopy image data sets produced in each case, respectively - which landmark is to be tapped next in succession and/or which fluoroscopy image is to be produced next in succession.

## Revendications

1. Procédé pour produire un modèle tridimensionnel d'une partie corporelle à l'aide d'un dispositif médical ou chirurgical de navigation et présentant les étapes suivantes :
- on relève et détermine la position de repères caractéristiques du modèle de partie corporelle à l'aide d'un appareil indicateur navigué ;
- on établit au moins deux ensembles de données d'image fluoroscopique respectivement pour une ou plusieurs régions de partie corporelle prédéterminées, individuelles et délimitées ;
- on détermine des données caractéristiques de partie corporelle à partir d'un traitement assisté par ordinateur et une combinaison des informations, notamment à partir des positions de repères déterminées directement et à partir de grandeurs caractéristiques qui ont été obtenues par traitement d'image à partir des ensembles de données fluoroscopiques ; et
- on établit, uniquement à partir des données caractéristiques de partie corporelle, un modèle tridimensionnel et déterminé dans sa position de partie corporelle pour la navigation chirurgicale assistée par imagerie.

2. Procédé selon la revendication 1, dans lequel les données caractéristiques de partie corporelle comprennent d'autres repères qui sont déterminés par calcul de symétrie sur des parties corporelles de forme symétrique ou sensiblement symétrique.

3. Procédé selon la revendication 1 ou 2, dans lequel les données caractéristiques de partie corporelle comprennent des longueurs de segment de partie corporelle et des angles de segment de partie corporelle les uns par rapport aux autres.

4. Procédé selon l'une quelconque des revendications 1 à 3, dans lequel on détermine en outre des centres de rotation articulés, en ce qu'on détermine la position de repères caractéristiques ou d'un agencement de références de navigation sur un os d'articulation, en plusieurs positions angulaires d'articulation, puis on recalcule le centre de rotation à partir des points de trajectoire obtenus.

5. Procédé selon l'une quelconque des revendications 1 à 4, dans lequel on produit un modèle de partie corporelle d'un os du fémur, et dans lequel on exécute une ou plusieurs des étapes suivantes :
- on relève et détermine la position des repères suivants avec l'appareil indicateur navigué :
-- épicondyle latéral,
-- épicondyle médial ;
- on détermine le centre de rotation articulé de l'os du fémur par :
-- détermination de la position de l'un des repères mentionnés ci-dessus ou d'un agencement de référence de navigation en plusieurs positions angulaires d'articulation et par re-calcul du centre à partir des points de trajectoire obtenus ; et/ou
-- un traitement d'image en utilisant des images fluoroscopiques qui sont prises en différentes positions angulaires d'articulation ;
- on détermine l'axe mécanique de l'os du fémur par une liaison du centre de rotation d'articulation avec le centre entre l'épicondyle latéral et l'épicondyle médial ;
- on détermine l'axe anatomique de l'os du fémur par un traitement d'image en utilisant des images fluoroscopiques de la zone de la tige de l'os et en déterminant plusieurs points d'axe au milieu de l'os qui définissent ensuite le parcours de l'axe anatomique ;
- on détermine l'axe du col par
-- un traitement d'image en utilisant deux images fluoroscopiques de la zone du col du fémur ; et/ou
-- un traitement d'image en utilisant une image fluoroscopique de la zone du col du fémur, et en utilisant la position du centre de rotation d'articulation ainsi que d'une position déversée connue ou prédéterminée de l'axe du col par rapport à l'axe anatomique ; et/ou
-- le relevé et la détermination de la position de points ou de centres sur le col du fémur ;
- on détermine l'antétorsion à partir d'informations déjà obtenues, en particulier à partir de la position de la liaison entre l'épicondyle latéral et l'épicondyle médial et de la position de l'axe mécanique.

6. Procédé selon l'une quelconque des revendications 1 à 5, dans lequel on produit un modèle de partie corporelle d'un bassin, et dans lequel on exécute les étapes suivantes :
- on relève et détermine la position d'au moins une épine iliaque antérieure supérieure avec l'appareil indicateur navigué ;
- on détermine le plan médial sagittal du bassin par un traitement d'image en utilisant des images fluoroscopiques d'une zone symétrique de la structure du bassin et des propriétés de symétrie du bassin, la position de la seconde épine iliaque antérieure supérieure pouvant aussi être déterminée ;
- on détermine le plan frontal du bassin par une détermination de position pour le pubis avant au moyen d'un traitement d'image en utilisant des images fluoroscopiques de la zone de joint du pubis et par liaison du point avec les points déterminés pour l'épine iliaque antérieure supérieure.

7. Procédé selon l'une quelconque des revendications 1 à 6, dans lequel on complète ou achève le modèle de partie corporelle à l'aide de données génériques de partie corporelle, en particulier par utilisation d'un modèle générique de partie corporelle qui a été adapté à l'aide d'informations déjà obtenues pour le modèle de partie corporelle à produire.

8. Procédé selon l'une quelconque des revendications 1 à 7, dans lequel avant de relever un repère ou d'établir les ensembles de données d'image fluoroscopique, on affiche sur une sortie image quel repère doit être relevé le premier par la suite ou quelle image fluoroscopique doit être réalisée en premier par la suite.
